# EUROPEAN PATENT SPECIFICATION

(11) **EP 1 455 842 B1**
(45) Date of publication and mention of the grant of the patent: **26.04.2006**
(21) Application number: 99949551.8
(22) Date of filing: 23.08.1999
(51) Int. Cl.: A61L 2/04, B27K 5/00, B63B 17/02

(54) **METHOD FOR WOOD CHIP PASTEURIZATION**
VERFAHREN ZUR PASTEURISIERUNG VON HOLZSPÄNEN
PROCEDE DE PASTEURISATION DE COPEAUX DE BOIS

(43) Date of publication of application: 15.09.2004
(73) Proprietor: Westward Corporation, Eugene, OR 97045 (US)
(72) Inventor: MEREDITH, Michael, D., Tigard, OR 97223 (US); TOMIC, Garth, L., Vancouver, WA 98668 (US)
(74) Representative: Fleuchaus, Leo
(86) International application number: PCT/US1999/019274
(87) International publication number: WO 2001/013961

(56) References cited:
- DE-A- 2 247 985
- DE-A- 2 431 742
- FR-A- 1 031 730
- US-A- 5 322 405
- US-A- 5 578 274

## Description

### BACKGROUND OF THE INVENTION

This invention relates to methods for ridding wood chips, particularly imported wood chips. of pathogens such as insects, nematodes and fungi. More particularly, the invention pertains to such methods employing hot water for pasteurization and for transport of the wood chips from a vessel, for example a ship, and an air circulation system circulation system to prevent escapement of pathogens.

Imported wood fiber of any sort and imported wood chips in particular, hereinafter referred to as "comminuted wood," pose a health threat to domestic plant species in potentially carrying pathogens from a site of origin to a site of delivery. Typically, poisonous chemicals such as aluminum phosphide or methyl bromide have been used for fumigation of imported wood fibers such as wood chips. However, such treatment chemicals cannot penetrate wood chips or large containers adequately within reasonable time. In addition, methyl bromide has been implicated by the Environmental Protection Agency in contributing to ozone depletion and its use is controversial. There is, generally, a conflict between the ability of a poison to function and to be harmless to the natural environment.

Attempts to employ a non-toxic and environmentally compatible agent for exterminating insects include utilizing an inert freezing liquid, such as liquid nitrogen, as proposed for example in Tallon, U.S. Patent No. 5,165,199. However, the method of Tallon is employed for relatively small areas and volumes, as liquid nitrogen can be prohibitively expensive in large quantities. Moreover, liquid nitrogen must generally be kept away from structures or articles that it is desired should not become brittle in view of stresses placed thereon. In addition, a freezing agent may not be effective against some pathogens, such as eggs, spores and seeds.

Pasteurization has long been used to treat food products, particularly milk products, and it has even been proposed in Roth et al., U.S. Patent No. 5,372,149 to pasteurize snuff. in the particular process of Roth, it is proposed to cook snuff in a steam jacketed vessel with stirring plows to bring the snuff into contact with the heated walls. While pasteurization has been employed in articles for human ingestion, it has apparently not been proposed to employ such a process in the treatment of wood chips. In particular, it has not been previously known how to apply such a process in the treatment of imported wood chips, which are generally delivered to a delivery site in extremely large quantities in a ship's cargo hold, where it is desirable to prevent the escape of pathogens to the local environment.
Accordingly, there is a need for a novel and improved method for wood chip pasteurization that pasteurizes wood chips prior to exposure to the local atmosphere and which is relatively inexpensive, non-toxic and environmentally compatible.

### SUMMARY OF THE INVENTION

The method for pasteurizing wood chips of the present invention solves the aforementioned problems and meets the aforementioned needs by employing a housing which is set upon and substantially seals the hatch of a ship's cargo hold in which wood chips are held, the housing being connected to an air removal and cleaning system, and employing an inlet to the hold for a liquid such as hot water and an outlet for a slurry, the slurry being a combination of the wood chips and the hot water.

The air removal and cleaning system controls the temperature within the ship's hold and provides fresh air so that a person may work therein to deliver the wood chips to the slurry conveying system. Preferably as well, the air removal and cleaning system maintains a slightly negative air pressure within the housing which, along with the seal, helps to prevent pathogens from escaping into the atmosphere.

The hot water outlet of the hot water system is fed into the inlet of the slurry conveying system into which wood chips are also fed by the human operator aided by a machines, e. g., a clam-shell or bucket, to create the slurry. The slurry is pumped through a sealed pipe feeding a remotely located pasteurization vessel wherein the slurry is deposited at the top and is pumped from the bottom. The slurry is caused to be held in the vessel at a predetermined minimum pasteurization temperature, determined by the hot water temperature, for a predetermined minimum pasteurization time. Subsequently, the slurry is pumped from the pasteurization vessel and drained, and the remaining wood chips are output from the system for collection and transport.

Water drained from the slurry is collected, reheated as necessary to reach the temperature required for the process, and recirculated to produce the slurry. Waste heat may be used to reheat the water. Therefore, it is a principal object of the present invention to provide a novel method for pasteurizing wood chips. It is another object of the present invention to provide such a method that isolates wood chips from the local atmosphere and pasteurizes the wood chips before exposure to the local atmosphere. It is still another object of the present invention to provide such a method that is relatively inexpensive. It is a further object of the present invention to provide such a method that does not rely on toxic substances. It is still a further object of the present invention to provide such a method that is environmentally compatible.

The foregoing and other objects, features and advantages of the present invention will be more readily understood upon consideration of the following detailed description of the invention, taken in conjunction with the following drawing.

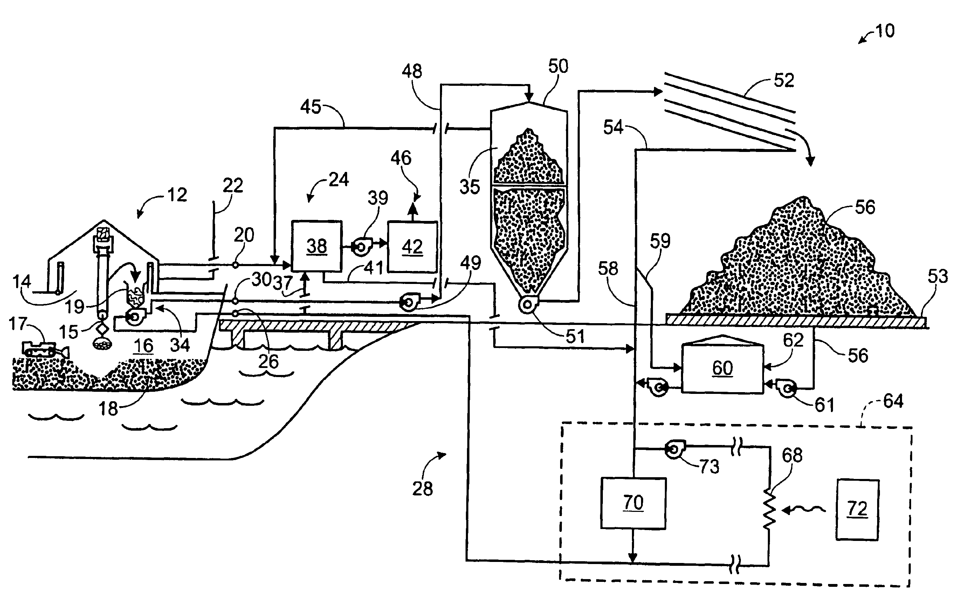

### BRIEF DESCRIPTION OF THE DRAWING

The Figure is a schematic of a method for pasteurizing wood chips according to the present invention.

### DETAILED DESCRIPTION OF A PREFERRED EMBODIMENT

Referring to the Figure, a method ("the system") for pasteurizing wood chips 18 is shown. The system pertains to pasteurizing wood chips that are held in the cargo hold 16 of a sea-going vessel. The vessel has an upper decking surface which has an opening therethrough for accessing the cargo hold. Around the opening is a hatch 14 which covers and protects the opening and is adapted to open upwardly (as shown).

The wood chips are held in the cargo hold to prevent the escape of pathogens to the external atmosphere. However, when opening the hatch at the port of entry of the vessel, the chips normally become exposed to the local atmosphere. To prevent this, a housing 12 is provided for removable placement over the opening and the hatch 14. Preferably, the housing is placed over the hatch at the site of delivery of the wood chips before opening the hatch, so that the wood chips may be rid of pathogens before being exposed to the local atmosphere. The housing sets on the upper decking surface as shown in the Figure. The housing is large enough to permit the hatch 14 its full range of opening when the housing is set thereover, and to provide room for workers and machinery, such as cranes and bulldozers, to fit inside and operate on the comminuted wood, to unload the wood from the vessel and treat it on shore as described more fully below.
During the shipping, micro-organisms acting on the wood chips 18 typically create an oxygen poor environment within the hold 16, in which the temperature is typically about 140 degrees Fahrenheit and the humidity is nearly 100 percent. It is desirable to neutralize this hostile environment to permit the workers to work within the housing and the hold. For this purpose, the housing is adapted for sealing around the hatch 14, through contact with the upper decking surface, to prevent the escape of pathogens from the hold.

In support of this purpose an air removal and cleaning system 24 is provided.
The housing 12 is connected to the air removal and cleaning system at output manifold 20. A filter 42 is provided for removing entrained organisms and particulate from the air. Fan 39 transfers the air through the filter 42. The air passing through the filter is the expelled into the atmosphere through an atmospheric air outlet 46. In addition, air removal from the hold 16 through the manifold 20 may be treated with water to remove a large portion of the particulate prior to the filtering. Hot water may be employed to pasteurize the airborne particulate. The air is transferred through a hot scrubber 38 to the filter 42, the hot scrubber contacting hot water with the air. Hot water is provided at inlet 37 and expelled at outlet 41 as described below. The air removal and cleaning system 24 maintains a slightly negative air pressure in the hold 16 to prevent the escape of pathogens into the atmosphere. The air removal and cleaning system 24 preferably also receives air from a pasteurizing vessel 50 via conduit 45 for cleaning air within the vessel. Although adequate fresh air for personnel working within the vessel would ordinarily leak into the vessel due to the negative pressure therein, an air inlet 22 may be provided to supply fresh air.

The hopper 19 is connected to an input manifold 26 of a hot water circulation system 28, the hot water being circulated through the hopper 19 and being output at a manifold 30 of the hot water circulation system 28.

The wood chips 18 are fed into the hopper 19 of the hot water circulation system 28 by an appropriate means, such as a bulldozer 17 and crane 15. The wood chips 18 combine with the hot water provided by the hot water circulation system 28 to form the slurry 34.

The slurry 34 is pumped through a sealed pipe 48 by pump 49 feeding a remotely located pasteurization vessel 50. The slurry is deposited at the top of the pasteurization vessel to form a bed of chips 35, which is pumped as a slurry from the bottom of the vessel 50 by a pump and discharge device 51, whereby the pasteurization vessel acts as a first-in-first-out ("FIFO") storage container.

The pasteurization vessel 50 maintains the hot slurry so that it is held at or above a predetermined minimum pasteurization temperature T1, the temperature T1 preferably being about 180 degrees Fahrenheit. Preferably, the temperature in the pasteurization vessel 50 is maintained passively, through the use of appropriate insulating material which contains the hear of the slurry. Moreover, the rate at which the slurry 34 is pumped through the pasteurization vessel 50 is controlled so that the time of transit of the slurry through the vessel 50 during which the temperature is at or above the temperature T1 is a predetermined minimum pasteurization time "t", the time "t" preferably being about 75 minutes.

Although only one pasteurization vessel 50 is shown, it is to be recognized that two or more such vessels may be used. In particular, multiple pasteurizing vessels may be used to avoid "rot holding"; that is, to ensure that the slurry flows uniformly through the pasteurization vessel or vessels.

Slurry 34 exiting the pasteurization vessel 50 is pumped downstream by the pump and discharge device 51 to a water separator 52 wherein most of the water is drained off at drain pipe 54. The remaining wet wood chips are placed in a chip pile 56 to await collection and transport.

Preferably, water from the system 10 is reclaimed for recycling through the system. Water drained from the water separator 52 is preferentially sent to the hearing system 64 via a stand pipe 58. Any excess water from the drain pipe 54 above what is immediately demanded by the process overflows through pipe 59 into a surge tank 60. Any depth of water below what is immediately demanded is made up from the surge tank 60.

Recirculated hot water is supplied to the scrubber 38 via inlet 37 and returned via outlet 41 to the stand pipe 58. Drainage and rain water from the chip pile 56 collected on pad 53, which may be made, e.g., of concrete or black top, are pumped to the surge tank 60 by pump 61. A fresh water inlet 62 is provided for replacing lost water, primarily as a result of evaporation from and absorption into the wood chips.

The hot water circulation system 28 also employs a (the) heating system 64 for heating the water to a predetermined temperature T2. The temperature T2 is high enough so that, combined with the chips, the resulting temperature of the mixture will be at a predetermined design temperature, e.g., about 188 degrees Fahrenheit. The water may be heated by a hot water heater 70.

The heating system 64 of the hot water circulation system 28 may employ waste heat. The waste heat may be obtained, for example, by a heat exchange, such as tubular coils 68, coupled to a supplementary heat source 72. In that event, the hot water heater 70 provides heat when sufficient waste heat is not available.

While the waste heat source 72 may be located some distance away, thereby requiring a pump 73, the water heater 70 is preferably located near the water separator 52. The stand pipe 58 is preferably high enough that water will flow by gravity through the water heater 70 and into the hopper 19.

It is to be recognized that, while a specific method for pasteurizing wood chips has been shown as preferred, other configurations could be utilized, in addition to configurations already mentioned, without departing from the principles of the invention. In particular, pasteurizing of wood chips may be accomplished without the air circulation system, and by employing other means of providing thereto and subsequently reclaiming therefrom a heated fluid for a predetermined time.

## Claims

1. A method for pasteurizing comminuted wood (18), comprising:
- combining a heated fluid with the comminuted wood at a first location (19) to form a slurry;
- pumping said slurry (34) from said first location to a second location (50);
- maintaining said slurry at said second location at or above a predetermined temperature for at least a predetermined time sufficient to pasteurize the comminuted wood; and
- thereafter separating said heated fluid from said slurry to recover the comminuted wood (18).

2. The method of claim 1, further comprising recycling said fluid obtained from said step of separating to form said slurry (34).

3. The method according to claim 1 for preventing the spread of pathogens from comminuted wood delivered in a shipping container (16) in which the comminuted wood (18) is isolated from the local atmosphere to substantially prevent the escape of pathogens therefrom, comprising
- mixing a heated liquid with the comminuted wood inside the shipping container to form a slurry (34) for transporting the comminuted wood to a location outside of the shipping container;
- flowing the slurry from the inside of the shipping container to the location while said slurry remains isolated from the local atmosphere to substantially prevent the escape of pathogens therefrom; and
- maintaining said slurry at or above a predetermined, elevated temperature for at least a predetermined time at said location to pasteurize the comminuted wood while said slurry remains isolated from the local atmosphere to substantially prevent the escape of pathogens therefrom.

4. The method of claim 3, wherein said liquid is water, the method further comprising heating said water prior to mixing with the comminuted wood (18).

5. The method of claim 3, further comprising providing a holding vessel (50) for maintaining said slurry (34) at said predetermined elevated temperature for said time at said location.

6. The method of claim 5, further comprising heating said liquid prior to mixing with the comminuted wood (18).

7. The method of claim 5, further comprising continuously forming and transporting said slurry (34) to said holding vessel (50) and continuously releasing said slurry from said holding vessel on a first-in-first-out basis to maintain said slurry at or above said predetermined elevated temperature for at least said predetermined time.

8. The method of claim 7, further comprising separating said liquid from said slurry (34) after releasing said slurry from said holding vessel (50), to recover the comminuted wood (18).

9. The method of claim 8, further comprising recycling said liquid after releasing said liquid from said slurry (34) to form said slurry.

10. The method of claim 9, further comprising heating said liquid prior to forming said slurry (34).

11. The method of claim 10, wherein said heating of said liquid is done by using waste heat obtained from other independent processes.

12. The method of claim 3, wherein the shipping container (16) has an opening, the method further comprising placing a cover over the opening and providing a liquid inlet and slurry (34) outlet from outside the container to inside the container.

13. The method of claim 12, wherein said cover comprises a housing (12) providing room for machinery and personnel to mix said liquid with the comminuted wood (18) within the container (16).

14. The method of claim13, further comprising allowing fresh air into said housing (12) and removing existing air from within said housing, filtering said existing air, and releasing said existing air into the local atmosphere.

15. The method of claim 14, further comprising removing said existing air from said housing (12) at a rate which maintains negative pressure within said housing.

16. Use of the method according to any of claims 1 to 15 for transporting comminuted wood (18) from a seagoing vessel to a receptacle on-shore and for pasteurizing the comminuted wood to substantially prevent the spread of pathogens to the local atmosphere, wherein
the sea-going vessel has an opening through an upper decking surface thereof, the opening providing access to a cargo hold (16) disposed beneath the upper decking surface, the opening being covered by a hatch door (14), a housing (12) is disposed over said opening and said hatch door (14), and on the upper decking surface of the sea-going vessel, said housing being adapted for sealing with the upper decking surface to substantially prevent the passage of air from the cargo hold to the external atmosphere.

17. The use according to claim 16, wherein said housing (12) is connected to an air outlet (46) for removing existing air from said cargo hold (16) through said outlet.

18. The use according to claim 17, wherein said outlet is part of an air removal and cleaning system which includes a hot scrubber in communication with said air outlet (46) for bringing said existing air in contact with a hot liquid.

19. The use of according to claim 16 wherein said housing (12) being further adapted so that said hatch door can be fully opened upwardly while said housing (12) is set on the upper decking surface, and the housing (12) is connected to an air outlet (46) for removing existing air from said cargo hold (16) through said air outlet.

20. The use according to claim 19, wherein said air outlet is part of an air transfer system which includes a filter (42) in communication with said air outlet.

21. The use according to claim 19, wherein said air outlet is pan of an air removal and cleaning system (24) which includes a hot scrubber (38) in communication with said air outlet for bringing said existing air in contact with a hot liquid.

## Patentansprüche

1. Verfahren zum Pasteurisieren von zerkleinertem Holz (18), das Folgendes umfasst:
- Kombinieren einer erhitzten Flüssigkeit mit dem zerkleinerten Holz an einem ersten Ort (19) zum Bilden eines Breis;
- Pumpen des Breis (34) von dem ersten Ort zu einem zweiten Ort (50);
- Beibehalten des Breis an dem zweiten Ort bei oder über einer vorbestimmten Temperatur während mindestens eines vorbestimmten Zeitraums, der ausreicht, um das zerkleinerte Holz zu pasteurisieren; und
- danach Trennen der erhitzten Flüssigkeit von dem Brei zum Zurückerlangen des zerkleinerten Holzes (18).

2. Verfahren nach Anspruch 1, das ferner die Wiederverwertung der von dem Schritt des Trennens erhaltenen Flüssigkeit zum Bilden des Breis (34) umfasst.

3. Verfahren nach Anspruch 1 zum Verhindern der Ausbreitung von Krankheitserregern von zerkleinertem Holz, das in einem Transportbehälter (16) geliefert wird, in dem das zerkleinerte Holz (18) von der lokalen Atmosphäre isoliert wird, um das Entweichen von Krankheitserregern davon im Wesentlichen zu verhindern, das Folgendes umfasst:
- Mischen einer erhitzten Flüssigkeit mit dem zerkleinerten Holz innerhalb des Transportbehälters zum Bilden eines Breis (34) zum Transportieren des zerkleinerten Holzes an einen Ort außerhalb des Transportbehälters;
- Fließen des Breis von innerhalb des Transportbehälters zum Ort, während der Brei von der lokalen Atmosphäre isoliert bleibt, um das Entweichen von Krankheitserregern davon im Wesentlichen zu verhindern; und
- Beibehalten des Breis bei oder über einer vorbestimmten hohen Temperatur während mindestens eines vorbestimmten Zeitraums an dem Ort zum Pasteurisieren des zerkleinerten Holzes während der Brei von der lokalen Atmosphäre isoliert bleibt, um das Entweichen von Krankheitserregern davon im Wesentlichen zu verhindern.

4. Verfahren nach Anspruch 3, wobei die Flüssigkeit Wasser ist und das Verfahren ferner das Erhitzen des Wassers vor dem Mischen mit dem zerkleinerten Holz (18) umfasst.

5. Verfahren nach Anspruch 3, das ferner das Bereitstellen eines Lagerbehälters (50) zum Beibehalten des Breis (34) bei der vorbestimmten hohen Temperatur während des Zeitraums an dem Ort umfasst.

6. Verfahren nach Anspruch 5, das ferner das Erhitzen der Flüssigkeit vor dem Mischen mit dem zerkleinerten Holz (18) umfasst.

7. Verfahren nach Anspruch 5, das ferner das kontinuierliche Bilden und Transportieren des Breis (34) zu dem Lagerbehälter (50) und das kontinuierliche Ablassen des Breis aus dem Lagerbehälter nach dem First-In-First-Out-Prinzip zum Beibehalten des Breis bei oder über der vorbestimmten hohen Temperatur während mindestens des vorbestimmten Zeitraums umfasst.

8. Verfahren nach Anspruch 7, das ferner, nach dem Ablassen des Breis aus dem Lagerbehälter (50), das Trennen der Flüssigkeit von dem Brei (34) zum Zurückerlangen des zerkleinerten Holzes (18) umfasst.

9. Verfahren nach Anspruch 8, das ferner die Wiederverwertung der Flüssigkeit nach dem Ablassen der Flüssigkeit von dem Brei (34) zum Bilden des Breis umfasst.

10. Verfahren nach Anspruch 9, das ferner das Erhitzen der Flüssigkeit vor dem Bilden des Breis (34) umfasst.

11. Verfahren nach Anspruch 10, wobei die Erhitzung der Flüssigkeit durch Verwendung von Abwärme, die von anderen unabhängigen Prozessen erhalten wird, ausgeführt wird.

12. Verfahren nach Anspruch 3, wobei der Transportbehälter (16) eine Öffnung hat, wobei das Verfahren ferner das Anordnen einer Abdeckung über der Öffnung und das Bereitstellen eines Flüssigkeitseinlasses und Breiauslasses (34) von außerhalb des Behälters in den Behälter umfasst.

13. Verfahren nach Anspruch 12, wobei die Abdeckung ein Gehäuse (12) umfasst, das Raum für Maschinen und Personal zum Mischen der Flüssigkeit mit dem zerkleinerten Holz (18) innerhalb des Behälters (16) bereitstellt.

14. Verfahren nach Anspruch 13, das ferner das Durchlassen frischer Luft in das Gehäuse (12) und das Entfernen bestehender Luft von innerhalb des Gehäuses, das Filtern der bestehenden Luft und das Ablassen der bestehenden Luft in die lokale Atmosphäre umfasst.

15. Verfahren nach Anspruch 14, das ferner das Entfernen der bestehenden Luft von dem Gehäuse (12) bei einer Rate, die negativen Druck innerhalb des Gehäuses beibehält, umfasst.

16. Verwendung des Verfahrens nach einem der Ansprüche 1 bis 15 zum Transportieren zerkleinerten Holzes (18) von einem Seeschiff zu einem Behälter an Land und zum Pasteurisieren des zerkleinerten Holzes, um die Ausbreitung von Krankheitserregern in die lokale Atmosphäre im Wesentlichen zu verhindern, wobei das Seeschiff eine Öffnung durch eine obere Deckoberfläche davon hat, wobei die Öffnung einen Zugang zu einem Frachtraum (16) bereitstellt, der unterhalb der oberen Deckoberfläche angeordnet ist, wobei die Öffnung durch eine Lukentür (14) bedeckt ist, ein Gehäuse (12) über der Öffnung und der Lukentür (14) angeordnet ist, und das Gehäuse an der oberen Deckoberfläche des Seeschiffs angepasst ist, um mit der oberen Deckoberfläche zu verschließen, um den Durchgang von Luft vom Laderaum zur externen Atmosphäre im Wesentlichen zu verhindern.

17. Verwendung nach Anspruch 16, wobei das Gehäuse (12) mit einem Luftauslass (46) zum Entfernen bestehender Luft von dem Laderaum (16) durch den Auslass verbunden ist.

18. Verwendung nach Anspruch 17, wobei der Auslass ein Teil eines Luftentfernungs- und Reinigungssystems ist, das einen heißen Skrubber in Verbindung mit dem Luftauslass (46) umfasst, um bestehende Luft in Kontakt mit einer heißen Flüssigkeit zu bringen.

19. Verwendung nach Anspruch 16, wobei das Gehäuse (12) ferner derart angepasst ist, dass die Lukentür nach oben vollständig geöffnet werden kann, während das Gehäuse (12) auf die obere Deckoberfläche gesetzt ist, und das Gehäuse (12) mit einem Luftauslass (46) zum Entfernen bestehender Luft aus dem Frachtraum (16) durch den Luftauslass verbunden ist.

20. Verwendung nach Anspruch 19, wobei der Luftauslass Teil eines Lufttransfersystems ist, das einen Filter (42) umfasst, der mit dem Luftauslass in Verbindung steht.

21. Verwendung nach Anspruch 19, wobei der Luftauslass Teil eines Luftentfernungs- und Reinigungssystems (24) ist, das einen heißen Skrubber (38) in Verbindung mit dem Luftauslass umfasst, um die bestehende Luft in Kontakt mit einer heißen Flüssigkeit zu bringen.

## Revendications

1. Un procédé pour pasteuriser du bois broyé (18) comprenant :
- combinaison d'un fluide chauffé avec le bois broyé à un premier emplacement (19) pour former une pâte liquide
- pompage de ladite pâte liquide (34) dudit premier emplacement vers un second emplacement (50)
- maintien de ladite pâte liquide au niveau dudit second emplacement à ou au-delà d'une température prédéterminée pour au moins une période de temps prédéterminée suffisante pour pasteuriser le bois broyé ; et
- après cela séparation dudit fluide chauffé de ladite pâte liquide pour récupérer le bois broyé (18)

2. Le procédé de la revendication 1, comprenant de plus le recyclage dudit fluide obtenu de ladite étape de séparation pour former ladite pâte liquide (34).

3. Le procédé selon la revendication 1 pour empêcher la dispersion d'agents pathogène du bois broyé livré dans une unité de transport (ou dans un emballage d'expédition) (16) dans lequel le bois broyé (18) est isolé de l'atmosphère locale pour substantiellement empêcher l'échappement d'agents pathogènes de là, comprenant
- mixage d'un fluide chauffé avec le bois broyé à l'intérieur de l'unité de transport pour former une pâte liquide (34) pour transporter le bois broyé vers un emplacement à l'extérieur de l'unité de transport ;
- écoulement de la pâte liquide de l'intérieur de l'unité de transport vers l'emplacement pendant que la pâte liquide reste isolée de l'atmosphère locale pour substantiellement empêcher l'échappement d'agents pathogène de là ; et
- maintien de ladite pâte liquide à ou au-delà d'une température élevée prédéterminée pour au moins une période de temps prédéterminée audit emplacement pour pasteuriser le bois broyé pendant que ladite pâte liquide reste isolée de l'atmosphère locale pour substantiellement empêcher l'échappement d'agents pathogène de là.

4. Le procédé selon la revendication 3 où ledit fluide est de l'eau, le procédé comprenant de plus le chauffage de ladite eau avant mixage avec le bois broyé (18).

5. Le procédé selon la revendication 3 comprenant de plus la fourniture d'un cuve support (50) pour maintenir ladite pâte liquide (34) à ladite température élevée pour ladite période de temps audit emplacement.

6. Le procédé selon la revendication 5, comprenant de plus le chauffage dudit fluide avant mixage avec le bois broyé (18).

7. Le procédé selon la revendication 5 comprenant de plus la formation continuelle et le transport de ladite pâte liquide (34) vers ledit cuve support (50) et le relâchement continuel de ladite pâte liquide dudit cuve support sur une base premier entré premier sorti pour maintenir ladite pâte liquide à ou au-delà d'une température élevée prédéterminée pour au moins ladite période de temps.

8. Le procédé selon la revendication 7 comprenant de plus la séparation dudit fluide de ladite pâte liquide (34) après avoir relâcher ladite pâte dudit cuve support (50) pour récupérer le bois broyé (18).

9. Le procédé selon la revendication 8 comprenant de plus le recyclage dudit fluide après relâchement/libération dudit fluide de ladite pâte liquide (34) pour former ladite pâte liquide.

10. Le procédé selon la revendication 9 comprenant de plus le chauffage dudit fluide avant formation de ladite pâte liquide (34).

11. Le procédé selon la revendication 10 où ledit chauffage dudit fluide est fait par utilisation de chaleur de récupération obtenue d'autres procès indépendants.

12. Le procédé selon la revendication 3 où l'unité de transport (16) a une ouverture, le procédé comprenant de plus le placement d'un couvercle sur l'ouverture et la fourniture d'une arrivée fluide et d'une sortie de pâte liquide (34) depuis l'extérieur de l'unité vers l'intérieur de l'unité.

13. Le procédé selon la revendication 12 où ledit couvercle comprenant un logement (12) fournissant de la place pour les machines et le personnel pour mixer ledit fluide avec le bois broyé (18) à l'intérieur de l'unité (16).

14. Le procédé selon la revendication 13 comprenant de plus le passage d'air frais à l'intérieur dudit logement (12) et la suppression de l'air existant de l'intérieur dudit logement, le filtrage de l'air existant, et le relâchement de l'air existant dans l'atmosphère local.

15. Le procédé selon la revendication 14 comprenant de plus la suppression de l'air existant dudit logement (12) à un taux qui maintient une pression négative à l'intérieur dudit logement.

16. Utilisation du procédé selon l'une quelconques des revendications 1 à 15 pour transporter du bois broyé (18) depuis un navire de haute mer vers un réservoir à terre et pour pasteuriser le bois broyé pour substantiellement empêcher la dispersion d'agents pathogène dans l'atmosphère locale, où
le navire de haute mer a une ouverture à travers une surface de pontage supérieure de là, l'ouverture fournissant un accès vers une cale à marchandise (16) disposée sous la surface de pontage supérieure, l'ouverture étant recouverte par une porte d'écoutille (14), un logement (12) est disposé sur ladite ouverture et ladite porte d'écoutille (14) et sur la surface de pontage supérieure du navire de haute mer, ledit logement étant adapté pour fermer avec la surface de pontage supérieure pour substantiellement empêcher le passage de l'air depuis la cale à marchandise vers l'atmosphère externe.

17. L'utilisation selon la revendication 16 où ledit logement (12) est connecté à une sortie d'air (46) pour supprimer l'air existant de ladite cale à marchandise (16) à travers ladite sortie.

18. L'utilisation selon la revendication 17 où ladite sortie est partie d'un système de désaérage et de nettoyage qui inclut un purificateur chaud en communication avec ladite sortie d'air (46) pour mettre ledit air existant en contact avec un fluide chaud.

19. L'utilisation selon la revendication 16 où ledit logement (12) étant de plus adapté de sorte que ladite porte d'écoutille peut être ouverte de manière montante pendant que ledit logement (12) est mis sur la surface de pontage supérieure et le logement (12) est connecté à une sortie d'air (46) pour supprimer l'air existant de ladite cale à marchandise (16) à travers ladite sortie.

20. L'utilisation selon la revendication 19 où ladite sortie d'air est partie d'un système de transfert d'ait qui inclut un filtre (42) en communication avec ladite sortie d'air.

21. L'utilisation selon la revendication 19 où ladite sortie d'air est partie d'un système de désaérage et de nettoyage (24) qui inclut un purificateur chaud (38) en communication avec ladite sortie d'air pour mettre ledit air existant en contact avec un fluide chaud.
